# EUROPÄISCHE PATENTSCHRIFT

(11) **EP 1 849 427 B1**
(45) Veröffentlichungstag und Bekanntmachung des Hinweises auf die Patenterteilung: **11.02.2009**
(21) Anmeldenummer: 07007882.9
(22) Anmeldetag: 18.04.2007
(51) Int. Cl.: A61B 19/00

(54) **Chirurgische Kopfklemme**
Surgical head clamp
Serre-tête chirurgical

(30) Priorität: 24.04.2006 DE 202006006734 U
(43) Veröffentlichungstag der Anmeldung: 31.10.2007
(73) Patentinhaber: Gottfried Storz, Medizintechnik GmbH & Co. KG, 78532 Tuttlingen (DE)
(72) Erfinder: Sörensen, Niels, Prof. Dr., 97070 Würzburg (DE); Ziaja, Hermann, 90559 Burgthann (DE); Kreidler, Winfried, 78532 Tuttlingen (DE)
(74) Vertreter: Neymeyer, Franz

(56) Entgegenhaltungen:
- EP-A1- 0 623 318

## Beschreibung

Die Erfindung betrifft eine chirurgische Kopfklemme bestehend aus einem im Wesentlichen U-förmigen Haltebügel mit zwei an den Enden eines Verbindungssteges in dessen Ebene zur gleichen Seite hin verlaufenden Halterschenkeln, deren Schenkelenden in zueinander koaxialen Lagern verstellbar gelagerte Dornhalter aufweisen, die mit einem oder mehreren Haltedornen versehen sein können, wobei ein erster Dornhalter schwenkbeweglich an einem um eine gemeinsame Lagerachse verstellbaren Lagerteil des einen Schenkelendes befestigt ist und ein zweiter Dornhalter mittels einer Gewindespindel in der Lagerbohrung des anderen Schenkelendes verstellbar ist.

Aus DE 694 11 621 T2 (= EP 0 623 318 B1) ist bereits eine Kopf- oder Schädelklemme der gattungsgemäßen Art bekannt, bei welcher der eine Halterschenkel einen zweiarmigen Dornhalter trägt, der mittels einer besonderen Lagervorrichtung in einer axialen Bohrung des Halterschenkels drehbar und in unterschiedlichen Drehlagen fixierbar ist. Der zweiarmige Dornhalter ist dabei in einer gabelartigen Haltevorrichtung um eine quer zur Lagerachse verlaufende Schwenkachse schwenkbar gelagert.

Ein dem zweiarmigen Dornhalter gegenüberliegender Einzeldorn ist in einer Gewindespindel gelagert, die koaxial zur Lagerachse des zweiarmigen Dornhalters im gegenüberliegenden Halterschenkel eingesetzt ist. Die Gewindespindel ist mittels eines als Rändelscheibe ausgebildeten Drehknopfes durch Drehung axial einstellbar, so dass der Abstand dieses Einzeldornes von den beiden Dornen des zweiarmigen Dornhalters veränderbar ist und auf den jeweils zu fixierenden Patientenkopf eingestellt werden kann. Dabei kann der Einzeldorn in der Gewindespindel in axialer Richtung federnd gelagert sein.

Bei dieser bekannten Kopfklemme ist der die beiden in einer gemeinsamen Ebene liegenden Halterschenkel verbindende Verbindungssteg zweiteilig ausgebildet und in seiner Erstreckungsrichtung verlängerbar bzw. verkürzbar, so dass eine grobe Abstandsveränderung zwischen.dem Einzeldorn und den beiden Dornen des zweiarmigen Dornhalters möglich ist.

An einem Teil des Verbindungssteges ist eine mit einem Zahnkranz versehene Halterung vorgesehen, durch welche die gesamte Kopfklemme an einem Gestell in verschiedenen Schwenklagen befestigt werden kann. Dabei verläuft die Achse des Zahnkranzes, um welche die gesamte Kopfklemme in der einen oder anderen Schwenkrichtung verschwenkt und fixiert werden kann, rechtwinklig zur gemeinsamen Lagerachse der Gewindespindel und des Lagers des zweiarmigen Dornhalters.

In der Praxis hat sich jedoch ergeben, dass zweiarmige Dornhalter auf der dem Einzeldorn gegenüberliegenden Kopfseite nicht für alle Anwendungsfälle optimal sind und dass auch die zweiteilige Ausbildung des Verbindungssteges, durch welche die grobe Abstandsverstellung vorgenommen werden kann, eine umständliche Handhabung mit sich bringt, die verbesserungsbedürftig ist.

Weitere Kopfklemmen sind aus US 3 835 861, US 4 169 478 und US 5 254 079 bekannt. Auch bei diesen ist auf der dem Einzeldorn gegenüberliegenden Seite jeweils nur ein zweiarmiger Dornhalter vorgesehen. Zur groben Abstandseinstellung ist der Verbindungssteg zweiteilig ausgebildet. Dabei ist jeweils ein Teil des Verbindungssteges mit einer sägezahnartigen Rastverzahnung versehen, in welche ein mit Rastzähnen versehenes Rastglied federnd lösbar rastend eingreift.
Bei all diesen Kopfklemmen ist der Dornhalter des Einzeldorns in Form eines gegen Federdruck verschiebbaren Kolbens im zylindrischen Hohlraum einer Gewindespindel gelagert und stirnseitig mit einer Steckbohrung zur Aufnahme des Einzeldorns versehen. Auf der dem Einzeldorn abgewandten Stirnseite ist eine Anschlagschraube in den Halterschaft eingeschraubt, deren Kopf mit einer Unterlegscheibe an der Stirnfläche eines gerändelten Griffteils anliegt. Eine axiale Schnellverstellung unter Aufhebung des Gewindeeingriffs ist dabei nicht möglich.

Aus DE 197 18 535 C2 und DE 20 2004 006 726 U1 sind Kopfklemmen bekannt, bei denen die Hohlspindeln, in denen die Dornhalter gegen Federdruck mittels Gewindeeingriffen axial verschiebbar gelagert sind, äußere Rastverzahnungen aufweisen, in welche lösbare Rastelemente fixierend eingreifen. Durch Lösen der Rastelemente werden die Hohlspindeln in ihren Lagerbohrungen frei verschiebbar.

Der Erfindung liegt die Aufgabe zugrunde, eine chirurgische Kopf klemme der eingangs genannten Art zu schaffen, die bei einfacherer Bauweise eine erleichterte Handhabung, insbesondere eine einfachere Einstellung und Fixierung des verstellbaren Dornhalters und bei schonender Einwirkung eine bessere Fixierung eines Patientenkopfes ermöglicht, wobei die Gewindespindel unter Aufhebung des Gewindeeingriffs im Halterschenkel frei verschiebbar sein soll.

Gelöst wird diese Aufgabe erfindungsgemäß dadurch, dass der zweite Dornhalter aus einem mit einer stirnseitigen Steckbohrung versehenen Führungsschaft besteht, der koaxial zur Lagerachse federnd in der Gewindespindel gelagert ist, und dass die mit einem Außengewinde versehene Gewindespindel in einer glatten zylindrischen Lagerbohrung des anderen Schenkelendes verstellbar gelagert ist, wobei das Außengewinde als Kugelgewinde mit einem kreissegmentartigen Profil ausgebildet ist, in welches als Eingriffselemente zwei Kugeln eingreifen, die in der Wandung der Lagerbohrung sich diametral gegenüberliegend, axial unbeweglich angeordnet und mittels eines verstellbaren Riegelhebels in dessen Sperrlage mit dem Kugelgewinde in Eingriff gehalten sind und die in einer anderen Lage des Riegelhebels außer Eingriff mit der Gewindespindel bringbar sind, um die Gewindespindel in ihrer Lagerbohrung frei verschiebbar zu machen.

Mit der erfindungsgemäßen Ausgestaltung ist es möglich, auf beiden Einspannseiten den jeweiligen Erfordernissen entsprechend einen zweiarmigen oder dreiarmigen Dornhalter auf einfache Weise einzusetzen und somit die jeweils optimale Einspannung des zu behandelnden Patientenkopfes zu erreichen. Zudem läßt sich die grobe Abstandseinstellung zwischen dem Einzeldorn und dem jeweils gegenüberliegenden, mit mehreren Dornen versehenen Dornhalter, wesentlich einfacher, bequemer und vor allem übersichtlicher einstellen, so dass die Handhabung insgesamt einfacher und sicherer ist als bei den bekannten Kopfklemmen, bei denen die beiden Bügelschenkel gegeneinander verstellbar sind.

Bei den bekannten Kopfklemmen, bei denen die hohle Gewindespindel mit einem Innengewinde der Lagerbohrung des Bügelschenkels in unlösbarem Eingriff steht, ist ein freies Verschieben der Gewindespindel nicht möglich. Die Gewindespindel kann lediglich durch Drehbewegungen wie eine Schraube axial verstellt werden.
Bei der erfindungsgemäßen Gestaltung des Gewindeeingriffs hingegen besteht die Möglichkeit, den Gewindeeingriff aufzuheben und die Gewindespindel gemeinsam mit dem Dornhalter wahlweise in Hublängen die jeweils dem einfachen oder mehrfachen der Steigung des Kugelgewindes entsprechen, in axialer Richtung beliebig zu positionieren. Bei verriegeltem Eingriff der Kugeln kann eine Feineinstellung durch Drehen der Gewindespindel vorgenommen werden. Auf einen Gewindeeingriff zwischen dem Dornhalter und der Gewindespindel kann verzichtet werden.

Durch die Ausgestaltung nach Anspruch 2 ist gewährleistet, daß die Gewindespindel nach jeder manuellen Grobverstellung zwangsläufig eine Axialposition einnimmt, in welcher die beiden Kugeln sofort eine korrekte Eingriffslage vorfinden, so dass eine Gewindebeschädigung beim Umstellen des Riegelhebels in seine Sperrlage mit Sicherheit vermieden werden.

Weitere vorteilhafte Ausgestaltungen der Erfindung sind Gegenstand der Unteransprüche 3 bis 12, wobei die Ausgestaltungen nach den Ansprüchen 6 und 7 auf einfache Weise und mit einfachen Mitteln eine Sicherung des Riegelhebels in seiner Sperrlage ermöglichen.

Anhand der Zeichnung wird im Folgenden ein Ausführungsbeispiel der Erfindung näher erläutert. Es zeigt:
- Fig. 1: eine Kopfklemme in 3D-Seitenansicht;
- Fig. 2: die beiden Halterschenkelenden mit ihren Lagerbohrungen im Schnitt;
- Fig. 3: in vergrößerter Darstellung den Riegelhebel aus Fig. 1 als Einzelteil mit Sperrschieber;
- Fig. 4: eine Gewindespindel mit einem Dornhalter im Schnitt;
- Fig. 5: den Dornhalter aus Fig. 4 als Einzelteil;
- Fig. 6: den Dornhalter aus Fig. 5 mit einem Dornträger im Schnitt;
- Fig. 7: in vergrößerter Darstellung den Endabschnitt der Gewindespindel aus Fig. 4;
- Fig. 8: im Schnitt einen zweiarmigen Dornträger mit seinen Befestigungsteilen in Explosionsdarstellung;
- Fig. 9: den zweiarmigen Dornträger in Stirnansicht;
- Fig. 10: den dreiarmigen Dornträger in Stirnansicht;
- Fig. 11: etwas vergrößert einen Bügelschenkel in Seitenansicht mit dem Riegelhebel und dessen Sicherungseinrichtung im Schnitt;
- Fig. 12: eine teilweise geschnittene Stirnansicht XII aus Fig. 11;
- Fig. 12a: den Riegelhebel aus Fig. 12 als Einzelteil in Frontansicht;
- Fig. 13: einen vergrößerten Ausschnitt aus Fig. 12 in Schnittdarstellung;
- Fig. 14: einen Ausschnitt aus Fig. 13 mit anderer Funktionslage des Riegelhebels und der Gewindekugeln.

Die in den Zeichnungsfiguren dargestellte Kopfklemme besteht aus einem U-förmigen Haltebügel 1, der an den Enden eines einteiligen Verbindungssteges 2 in dessen Ebene zur gleichen Seite hin verlaufende Halterschenkel 3 und 4 aufweist.

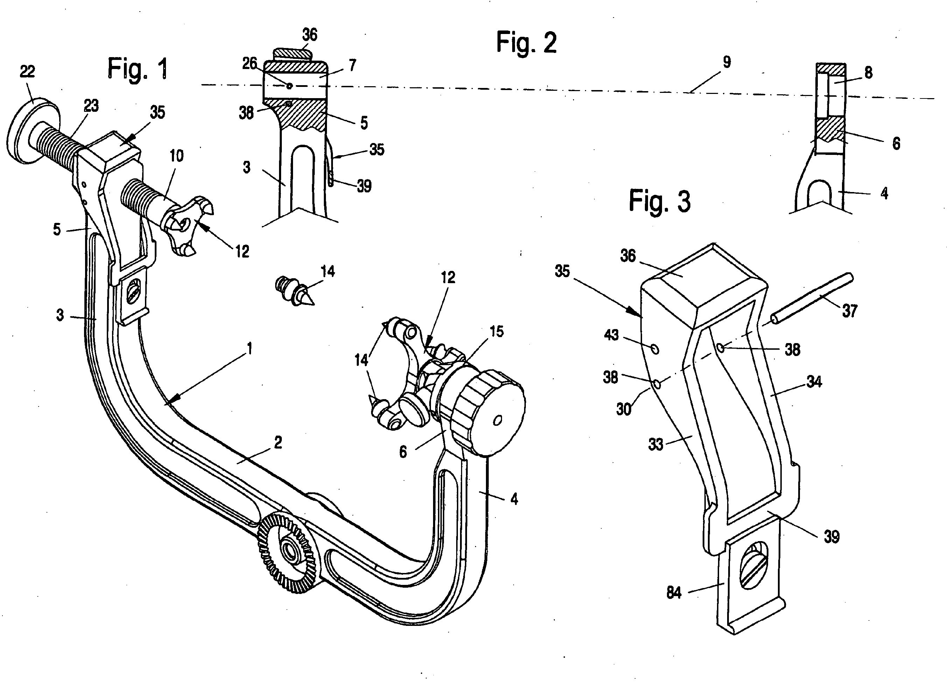

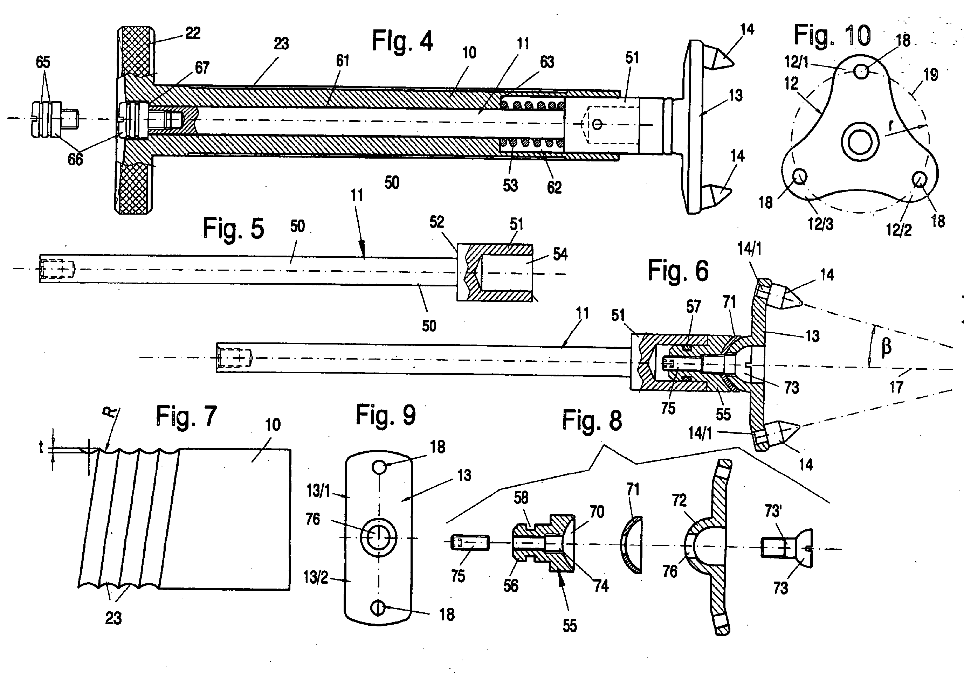

Die oberen Enden 5 und 6 der Halterschenkel 3 und 4 sind jeweils mit zueinander koaxialen Lagerbohrungen 7 bzw. 8 versehen, die eine gemeinsame Lagerachse 9 aufweisen (siehe Fig. 2). Die Lagerbohrung 7 dient zur Aufnahme und Führung einer Gewindespindel 10, in der ein Dornhalter 11 (Fig. 4 bis 6) gelagert ist. Dieser Dornhalter 11 ist in der in Fig. 1 dargestellten Ausführung mit einem dreiarmigen Dornträger 12 versehen. Er kann jedoch, wie nachstehend noch näher beschrieben ist, wahlweise mit einem zweiarmigen Dornträger 13 oder mit einem einfachen zur Lagerachse 9 koaxialen Haltedorn 14 versehen werden.

Die gegenüberliegende Lagerbohrung 8 des anderen Halterschenkels 4 dient der Lagerung eines Lagerteils 15, an dem ebenfalls wahlweise ein dreiarmiger Dornträger 12 oder ein zweiarmiger Dornträger 13 befestigbar ist.

Das Lagerteil 15 ist in der Lagerbohrung 8 mittels einer besonderen Lagervorrichtung um die Lagerachse 9 schwenkbar und in unterschiedlichen Winkelstellungen fixierbar.

Während der zweiarmige Dornträger 13 zwei symmetrisch sich diamtral gegenüber stehende Arme 13/1 und 13/2 mit jeweils nach innen geneigten Enden aufweist, besitzt der dreiarmige Dornhalter 12, wie aus Fig. 10 ersichtlich ist, drei Arme 12/1, 12/2 und 12/3, die gleiche Winkelabstände von jeweils 120° voneinander haben und deren Steckbohrungen 18 auf einem konzentrischen Kreis 19 mit dem Radius r liegen. Dabei sind die äußeren Abschnitte Arme 12/1, 12/2 und 12/3 ebenfalls zu einer zentralen Mittelachse hin gleichmäßig geneigt.

Da die Achsen der Haltedorne 14 mit den Achsen ihrer Steckbohrungen 18 zusammenfallen, schneiden sich auch die Achsen der Haltedorne 14 im gleichen Punkt auf der Mittelachse, die in einer Normallage des dreiarmigen Dornhalters 12 mit der Lagerachse 9 zusammenfällt.

Die Haltedorne 14, welche die Verbindung zum festzuhaltenden Patientenkopf herstellen, sind wie üblich mit einem in eine Steckbohrung 18 passenden Steckzapfen 14/1 versehen (Fig. 6).

Auch die Haltedorne 14 des zweiarmigen Dornträgers 13 sind zu dessen Mittelachse hin symmetrisch geneigt, so dass ihre Achsen 16 mit dieser Mittelachse 17 einen spitzen Winkel bilden (Fig. 6).

Der Dornhalter 11 besteht aus einem zylindrischen Führungsschaft 50 und einem im Durchmesser vergrößerten Kopfteil 51, der eine Anschlagschulter 52 für eine Druckfeder 53 bildet. Das Kopfteil 51 weist eine stirnseitig offene Steckbohrung 54 auf, die zur reibschlüssigen Aufnahme des Steckbolzens 55 dient, an dem wahlweise ein zweiarmiger oder dreiarmiger Dornträger 13 bzw. 12 befestigt werden kann.

Der Steckbolzen 55 ist mit einem im Durchmesser verjüngten Steckzapfen 56 versehen, der zur Erhöhung der Reibschlüssigkeit in einer Ringnut 58 einen elastischen, vorzugsweise aus Gummi oder Kunststoff bestehenden O-Ring 57 aufweist.

Die nach außen gerichtete Stirnfläche des Steckbolzens 55 ist mit einer kalottenförmigen Ausnehmung 70 versehen. Unter Zwischenlage einer gelochten, ebenfalls kalottenförmigen Kunststoffscheibe ist in der Ausnehmung 70 eine halbkugelförmige Kalottenwand 72 eines Dornträgers 13 oder 12 schwenkbeweglich und drehbar gelagert und mittels einer Kugelkopfschraube 73 mit dem Steckbolzen 55 verbunden. Die Kugelkopfschraube 73 ist, wie in Fig. 6 dargestellt, in eine zentrale Gewindebohrung 74 des Steckbolzens 55 eingeschraubt und mittels einer Konterschraube 75 in einer Axiallage so fixiert, daß der Dornträger 12 bzw. 13 in seiner jeweiligen Schwenkstellung reibungsschlüssig gehalten wird, sich aber manuell bzw. beim Ansetzen an einen Patientenkopf leicht verschwenken und drehen läßt. Um eine ausreichend große Verschwenkbarkeit des Dornträgers 12, 13 zu gewährleisten, ist die Kalottenwand 72 mit einer zentralen Bohrung 76 versehen, deren Durchmesser größer ist als der Gewindeschaft 73' der Kugelkopfschraube 73.

Wie aus Fig. 4 ersichtlich ist, weist die Gewindespindel 10 eine zentrale Axialbohrung 61 auf, in welcher der Führungsschaft 50 des Dornhalters 11 axial beweglich geführt ist. Zur axial beweglichen Aufnahme und Führung des im Durchmesser größeren Kopfteils 51 ist eine erweiterte koaxialer Aufnahmebohrung 62 vorgesehen, die mit der Axialbohrung 61 eine Ringschulter 63 bildet. Zwischen dieser Ringschulter 63 und der Anschlagschulter 52 des Kopfteils 51 befindet sich die einen Teil des Führungsschaftes 50 umschließende Druckfeder 53, die beim Einspannen eines Patientenkopfes einen gewissen Federhub zuläßt. Wie stark dabei die Druckfeder 53 belastet wird, ist an den Umfangsrillen 65 einer Schaftschraube 66 ablesbar, die stirnseitig in den Führungsschaft 50 eingeschraubt ist und zugleich als Bewegungsbegrenzer dient, wenn sie an einer hinteren Ringschulter 67 der Axialbohrung 61 anliegt.

Um einen Patientenkopf zwischen dem im Dornhalter 11 befestigten Dornträger 12 oder 13 und den Haltedornen 14 des gegenüberliegenden dreiarmigen Dornträgers 12 fest einspannen zu können, muß der Dornhalter 11 in der Lagerachse 9 verstellbar sein. Zu diesem Zweck ist nicht nur der Dornhalter 11 axial federnd in der Gewindespindel 10 gelagert, sondern es ist auch die mit dem gerändelten Handgriff 22 versehene Gewindespindel 10 außenseitig mit einem Kugelgewinde 23 versehen und axial verstellbar in der Lagerbohrung 7 des Halterschenkels 3 gelagert. Diese Lagerbohrung 7 hat eine glatte, zylindrische Innenfläche, so dass das Kugelgewinde mit der Lagerbohrung 7 nicht Eingriff steht, sondern darin frei verschiebbar ist.

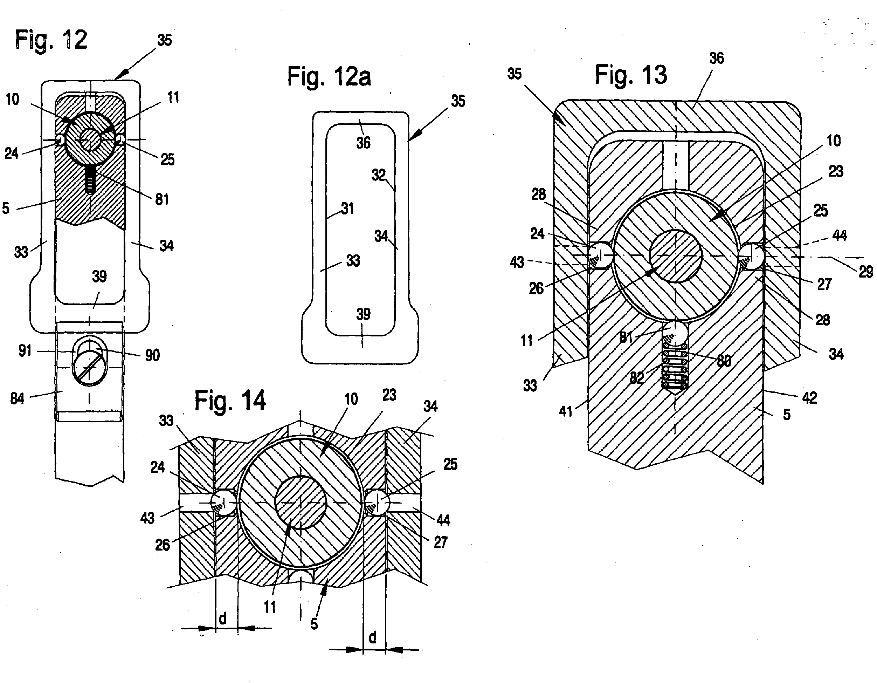

Mit dem Kugelgewinde 23 stehen jedoch als Eingriffselemente zwei gleiche Kugeln 24, 25 in Eingriff, die in zwei in Höhe der Lagerachse 9 sich diametral gegenüberliegenden Radialbohrungen 26 und 27 radial lose jedoch in axialer Richtung der Lagerachse 9 unbeweglich gelagert sind. Diese Radialbohrungen 26, 27 haben eine gemeinsame Achse 29, und sie befinden sich in der die Lagerbohrung 7 umfassenden Bohrungswand 28. Diese Bohrungswand 28 weist im Bereich dieser Radialbohrungen 26, 27 eine Dicke **d** (Fig. 14) auf, die kleiner ist als der Durchmesser der Kugeln 24, 25. Beim Ausführungsbeispiel haben die Kugeln 24, 25 einen Durchmesser von 3 mm, während die Wanddicke **d** 2,7 mm beträgt.

Es versteht sich, dass das Profil des Kugelgewindes 23 auf den Kugeldurchmesser abgestimmt ist, so dass sich die Gewindespindel 10 leicht drehen und dabei je nach Drehrichtung in der Lagerbohrung 7 kontrolliert verschieben läßt, wenn sich die Kugeln 24, 25 mit ihr im Eingriff befinden.

Der Profilradius R des im Profil kreissegmentförmigen Kugelgewindes 23 beträgt 1,5 mm und entspricht somit dem halben Kugeldurchmesser. In Eingriff gehalten mit dem Kugelgewinde 23 werden die Kugeln 24, 25 durch zwei planparallele Innenflächen 31, 32 der Gabelschenkel 33, 34 eines Riegelhebels 35.

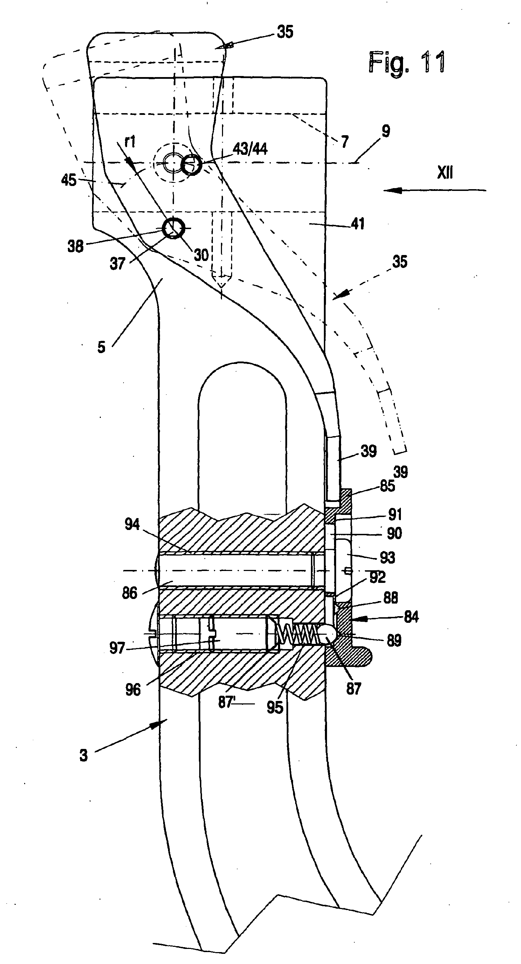

Der Halterschenkel 3 weist an seinem oberen Endabschnitt, in dessen Bereich auch die beiden Radialbohrungen 26, 27 liegen, zwei zur gemeinsamen Achse 29 dieser beiden Radialbohrungen 26, 27 rechtwinklig verlaufende, ebene äußere Begrenzungsflächen 40, 41 auf, an denen die Gabelschenkel 33, 34 mit ihren ebenfalls planebenen Innenflächen 31, 32 spielfrei anliegen. Durch diesen Innenflächen 31, 32 sind die Radialbohrungen 26, 27 jeweils in den Ebenen der Begrenzungsflächen 40 bzw. 41 abdeckbar. Der Riegelhebel 35 ist um eine zur Achse 29 der Radialbohrungen 26, 27 parallele Schwenkachse 30 (Fig. 3 und 11) schwenkbar am Halterschenkel 5 gelagert. Als Lagerelement dient ein Lagerzapfen 37, der im Halterschenkel 3 befestigt ist und in Lagerbohrungen 38 der beiden Gabelschenkel 33 und 34 ragt. Die beiden Gabelschenkel 33 und 34 sind oberseitig durch einen plattenartigen horizontalen Quersteg 36 und an ihren unteren Enden durch einen vertikalen Quersteg 39 miteinander starr verbunden, so dass sie nur gemeinsam betätigt werden können.

Die nach unten verlängerten und durch den Quersteg 39 miteinander verbundenen Endabschnitte der Gabelschenkel 33, 34 bilden gemeinsam einen Handgriff, mit welchem der Riegelhebel 35 leicht betätigt werden kann.

Wenn die beiden planparallelen Innenflächen 31 und 32 der Gabelschenkel 33, 34 des Riegelhebels 35 die Radialbohrungen 26, 27 abdecken, haben die beiden als Eingriffselemente dienenden Kugeln 24, 25 keine Möglichkeit, radial nach außen auszuweichen. Sie befinden sich dann formschlüssigem Zwangseingriff mit dem Kugelwinde 23 und fixieren damit die Gewindespindel 10 in der Lagerbohrung 7. In dieser Sperrstellung des Riegelhebels 35 und der Kugeln 24, 25 kann somit die Gewindespindel 53 in axialer Richtung nicht frei, sondern nur durch Drehen mehr oder weniger langsam bewegt werden.

Um den Kugeln 24, 25 die Möglichkeit zu geben, in radialer Richtung innerhalb der Radialbohrungen 26, 27 auszuweichen und eine freie axiale Verstellbewegung der Gewindespindel 10 zu ermöglichen, weisen die beiden Gabelschenkel 33 und 34 des Riegelhebels 35 Bohrungen 43 und 44 auf, die auf einem zu ihrer Schwenkachse 30 konzentrischen Kreisbogen 45 liegen und miteinander fluchten. Diese Bohrungen 43, 44 haben einen kleineren Durchmesser als die Kugeln 24, 25 und sind so ausgebildet, dass die Kugeln 24, 25 soweit radial in sie eindringen können, dass sie außer Eingriff mit dem Kugelgewinde 23 der Gewindespindel 10 gelangen können. D.h. die Kugeln 24, 25 können zur Freigabe der Gewindespindel 10 teilweise in die Bohrungen 43, 44 eintauchen. Der Radius rl des Kreisbogens 45 (Fig. 11) entspricht dabei exakt dem Abstand der Bohrungen 43, 44 von der Schwenkachse 30 des Riegelhebels 35. bei Ausführungsbeispiel haben die Kugeln 23, 24 einen Durchmesser von 3 mm und die Bohrungen 43, 44 einen Durchmesser von 1,8 mm.

Wie aus den Fig. 12 und 13 ersichtlich ist, befindet sich in der Lagerbohrung 7 in einer vertikal und radial verlaufenden Sackbohrung 80 eine Rastkugel 81, die gleich groß ist wie die Kugeln 23, 24. Diese Rastkugel 81 wird unter dem Einfluß einer Druckfeder 82 mit dem Kugelgewinde 23 der Gewindespindel 10 federnd in Eingriff gehalten. Gegenüber den Kugeln 24, 25 ist diese Rastkugel 81 in Umfangsrichtung um 90° und in axialer Richtung der Lagerachse 9 um ein Viertel der Gewindesteigung versetzt. Damit wird sichergestellt, dass die Gewindespindel 10 nach jeder freien Längsverschiebung sofort eine Axiallage einnimmt, in welcher die beiden Kugeln 24, 25 mit ihrem Kugelgewinde korrekt in Eingriff gelangen können und der Riegelhebel 35 ebenfalls sofort in sein Sperrlage gebracht werden kann. Dadurch wird die Gefahr einer mechanischen Beschädigung des Kugelgewindes 23 vermieden.

Um verhindern zu können, dass sich der Riegelhebel 35 ungewollt aus seiner in Fig. 11 in ausgezogenen Linien dargestellten Sperrlage, in welcher er die beiden Kugeln 24, 25 mit dem Kugelgewinde 23 in Eingriff hält, verschwenken und die Verriegelung aufheben kann, ist der Riegelhebel 35 in seiner die Radialbohrung 26, 27 abdeckenden Sperrlage arretierbar. Zu diesem Zweck ist an der Innenseite des Halterschenkels 3 ein Sperrschieber 84 angeordnet, der in seiner in Fig. 11 dargestellten Sperrlage mit einer Sperrzunge 85 den unteren Quersteg 39 des Riegelhebels 35 fixiert. Dieser Sperrschieber 84 ist mittels einer Schraube 86 am Halterschenkel 3 vertikal verschiebbar gelagert und mittels einer gefederten Rastkugel 87 einerseits in der gezeichneten Sperrlage und andererseits in einer nach unten versetzten unwirksamen Lage rastend arretierbar. Zu diesem Zweck ist der Sperrschieber 84 mit zwei Rastkerben 88 und 89 versehen, in welche die Rastkugel 87 wechselweise rastend eingreifen kann.
Der Sperrschieber 84 ist zur Aufnahme des Gewindeschaftes der Schraube 86 mit einem vertikalen Langloch 90 versehen, auf dessen versenktem Rand 91 die planebene Ringfläche 92 des Schraubenkopfes 93 führend aufsitzt.

Die Schraube 86 ist in eine horizontale Gewindebohrung 94 des Bügelschenkels 3 eingeschraubt. Die Rastkugel 87 liegt zusammen mit ihrer Druckfeder 87' in einer dazu parallelen Bohrung 95, an die sich eine Gewindebohrung 96 anschließt. In dieser Gewindebohrung 96 befindet sich eine Stellschraube 97, an der sich die Druckfeder 87' der Rastkugel 87 abstützt.

Zum Lösen des Riegelhebels 35 wird der Sperrschieber 84 nach unten verschoben bis die Rastkugel 87 in die obere Rastkerbe 88 einrastet. Der untere Quersteg 39 des Riegelhebels 35 ist dann frei und der Riegelhebel 35 kann in die in Fig. 11 in Phantomlinien dargestellte Lage verschwenkt werden, in welcher die beiden Bohrungen 43 und 44 mit den Kugeln 24, 25 fluchten. Dadurch wird der Zwangseingriff mit dem Kugelgewinde aufgehoben und die Gewindespindel 10 in der Lagerbohrung 7 frei bzw. unter Überwindung der Rastwirkung Rastkugel 81 verschiebbar. Es kann auf diese Weise eine axiale Grobeinstellung der Gewindespindel 10 mit dem Dornhalter 11 vorgenommen werden. Nach einer solchen Grobeinstellung wird der Riegelhebel 35 wieder in seine Sperrlage gebracht und mit dem Sperrschieber verriegelt. Die Kugeln 24, 25 befinden sich dann wieder im Zwangseingriff mit dem Kugelgewinde 23. Durch Drehen läßt sich dann die Gewindespindel 10 exakt in die richtige Spannposition bringen.

## Patentansprüche

1. Chirurgische Kopfklemme bestehend aus einem im wesentlichen U-förmigen Haltebügel (1) mit zwei an den Enden eines Verbindungssteges (2) in dessen Ebene zur gleichen Seite hin verlaufenden Halterschenkeln (3, 4), deren Schenkelenden (5, 6) in zueinander koaxialen Lagern verstellbar gelagerte Dornhalter (11) aufweisen, die mit einem oder mehreren Haltedornen (12) versehen sein können, wobei ein erster Dornhalter schwenkbeweglich an einem um eine gemeinsame Lagerachse (9) verstellbaren Lagerteil (15) des einen Schenkelendes (6) befestigt ist und ein zweiter Dornhalter (11) mittels einer Gewindespindel (10) in einer Lagerbohrung (7) des anderen Schenkendes (5) verstellbar ist,
**dadurch gekennzeichnet,**
**dass** der zweite Dornhalter (11) aus einem mit einer stirnseitigen Steckbohrung (54) versehenen Führungsschaft (50) besteht, der koaxial zur Lagerachse (9) federnd in einer Axialbohrung (61) der Gewindespindel (10) gelagert ist, und dass die mit einem Außengewinde versehene Gewindespindel(10) in einer glatten zylindrischen Lagerbohrung (7) des anderen Schenkelendes (3) verstellbar gelagert ist, wobei das Außengewinde als Kugelgewinde (23) mit einem kreissegmentartigen Profil ausgebildet ist, in welches als Eingriffselemente zwei Kugeln (24, 25) eingreifen, die in der Wandung (28) der Lagerbohrung (7) sich diametral gegenüberliegend, axial unbeweglich angeordnet und mittels eines verstellbaren Riegelhebels (35) in dessen Sperrlage mit dem Kugelgewinde (23) in Eingriff gehalten sind und die in einer anderen Lage des Riegelhebels (35) außer Eingriff mit der Gewindespindel (10) bringbar sind, um die Gewindespindel (10) in ihrer Lagerbohrung (7) frei verschiebbar zu machen.

2. Chirurgische Kopfklemme nach Anspruch 1, **dadurch gekennzeichnet, dass** die Lagerbohrung (7) zusätzlich mit einer radial federnd in das Kugelgewinde (23) eingreifenden Rastkugel (81) versehen ist.

3. Chirurgische Kopfklemme nach Anspruch 1, **dadurch gekennzeichnet, dass** die Kugeln (24, 25) jeweils in einer von zwei zueinander koaxialen Radialbohrungen (26, 27)) von Wandabschnitten (28) der die Gewindespindel (10) passend aufnehmenden Lagerbohrung (7) gelagert sind, wobei die Dicken (d) der Wandabschnitte (28) jeweils kleiner sind als der Kugeldurchmesser.

4. Kopfklemme nach Anspruch 2, **dadurch gekennzeichnet, dass** der Halterschenkel (3) wenigstens im Bereich der Radialbohrungen (26, 27) zwei zu deren Achse (29) rechtwinklig verlaufende, planparallele, ebene Außenflächen (41, 42) aufweist, an welchen schwenkbeweglich ein zweischenkliger Riegelhebel (35) angeordnet ist, durch dessen Gabelschenkel (33, 34) die Radialbohrungen (26, 27) jeweils in der Ebene einer der Begrenzungsflächen (41, 42) abdeckbar sind.

5. Kopfklemme nach Anspruch 4, **dadurch gekennzeichnet, dass** der Riegelhebel (35) zwei Gabelschenkel (33, 34) mit planparallelen Innenflächen (31, 32) aufweist, die an den Außenflächen (41, 42) des Halterschenkels (3) anliegen und die am Halterschenkel (3) um eine zur Achse (29) der Radialbohrungen (26, 27) parallele, exzentrische Schwenkachse (30) schwenkbar gelagert sind.

6. Kopf klemme nach Anspruch 5, **dadurch gekennzeichnet, dass** der Riegelhebel (35), dessen Gabelschenkel (33, 34) durch einen oberhalb des Halterschenkels (3) liegenden Quersteg (36 miteinander verbunden sind, mit einem Griffteil (39) versehen ist, der in der Sperrlage des Riegelhebels (35) mittels einer Sicherungseinrichtung (84) fixierbar ist.

7. Kopfklemme nach Anspruch 6, **dadurch gekennzeichnet, dass** der Griffteil als ein die Enden der Gabelschenkel (33, 34) verbindender Quersteg (39) ausgebildet ist, in dessen Bewegungsbahn ein Sperrschieber (84) der Sicherungseinrichtung sperrend einführbar ist.

8. Kopf klemme nach Anspruch 4 oder 5, **dadurch gekennzeichnet, dass** die beiden Gabelschenkel (33, 34) des Riegelhebels (35) auf einem zu ihrer Schwenkachse (30) konzentrischen Kreisbogen (45) liegende, miteinander fluchtende Ausnehmungen (43, 44) aufweisen, in welche die Kugeln (24,25) zur Freigabe der Gewindespindel (10) teilweise eintauchen können.

9. Kopfklemme nach Anspruch 8, **dadurch gekennzeichnet, dass** die Ausnehmungen aus Bohrungen (43, 44) bestehen, deren Durchmesser kleiner ist als der Durchmesser der unter sich gleichen Kugeln (24, 25).

10. Chirurgische Kopfklemme nach Anspruch 1, **dadurch gekennzeichnet, dass** sich die Steckbohrung (54) in einem eine radiale Ringschulter (52) für eine Druckfeder (53) bildenden Kopfteil (51) des Führungsschaftes (50) befindet, dem eine radiale Ringschulter (63) einer Axialbohrung (61) der Gewindespindel (10) axial gegenüber steht und daß der Führungsschaft (50) an seinem der Aufnahmebohrung gegenüber liegenden Ende mit einem seine federnde Axialbewegung begrenzenden Anschlagelement (66) versehen ist.

11. Chirurgische Kopfklemme nach Anspruch 1 oder 10, **dadurch gekennzeichnet, dass** zur Lagerung eines mit mehreren Haltedornen (14) versehenen mehrarmigen Dornträgers (12, 13) am Führungsschaft (50) ein mit einem in die Steckbohrung (54) passenden Steckansatz (56) versehener Steckbolzen (55) vorgesehen ist, der stirnseitig eine kalottenartige Vertiefung (70) zur schwenkbaren und drehbaren Lagerung eines mit einem passenden Gegenprofil versehenen mehrarmigen Dornträgers (12, 13) aufweist.

12. Chirurgische Kopfklemme nach Anspruch 11, **dadurch gekennzeichnet, dass** zur schwenkbaren Befestigung des mehrarmigen Dornträgers (12, 13) eine zum Gegenprofil passende Kugelkopfschraube (73) in eine zentrale Gewindebohrung (74) des Steckbolzens (55) eingeschraubt und mittels einer Konterschraube (75) fixiert ist.

## Claims

1. Surgical head clamp consisting of a substantially u-shaped holding frame (1) with two holding arms (3, 4) running from the ends of a connecting piece (2) in the same plane to the same side, the ends of the arms (5, 6) having pin holders (11) mounted adjustably in mutually coaxial bearings, which can be provided with one or more holding pins (12), whereby a first pin holder is secured pivotably on a bearing element (15) of one arm end (6), which is adjustable about a common bearing axis (9), and a second pin holder (11) can be adjusted by means of a threaded spindle (10) in a bearing bore (7) of the other arm end (5),
**characterised in that**
the second pin holder (11) consists of a guiding shaft (50) provided with a face end insertion bore (54), which shaft is mounted coaxially in relation to the bearing axis (9) and flexibly in an axial bore (61) of the threaded spindle (10), and **in that** the threaded spindle (10) provided with an external thread is mounted adjustably in a smooth cylindrical bearing bore (7) of the other arm end (3), whereby the external thread is in the form of a spherical thread (23) with a circular-segment-like profile, into which two balls (24, 25) engage as gripping elements, which are arranged in the wall (28) of the bearing bore (7) diametrically opposite one another and secured to be fixed axially, and by means of an adjustable locking lever (35) are held in a locked position by the spherical thread (23), and which can be moved into a different position of the locking lever (35) out of engagement with the threaded spindle (10) to make the threaded spindle (10) freely displaceable in its bearing bore (7).

2. Surgical head clamp according to claim 1, **characterised in that** the bearing bore (7) is additionally provided with a detent ball (81) which engages radially flexibly with the spherical thread (23).

3. Surgical head clamp according to claim 1, **characterised in that** the balls (24, 25) are mounted respectively in a bearing bore (7) receiving two coaxial radial bores (26, 27) of wall sections (28) of the bearing bore (7) receiving the threaded spindle (10), whereby the respective thicknesses (d) of the wall sections (28) are smaller than the sphere diameter.

4. Head clamp according to claim 2, **characterised in that** the holding arm (3) has, at least in the region of the radial bores (26, 27), two plane-parallel flat outer faces (41, 42) running at right angles to the axis (29) of the radial bores, on which outer faces a two-armed locking lever (35) is arranged pivotably and by means of the fork arms (33, 34) the radial bores (26, 27) can be covered in the plane of one of the boundary faces (41, 42).

5. Head clamp according to claim 4, **characterised in that** the locking lever (35) comprises two fork arms (33, 34) with plane-parallel inner faces (31, 32), which bear against the outer faces (41, 42) of the holding arm (3), and which are mounted pivotably on the holding arm (3) about an eccentric pivot axis (30) that is parallel to the axis (29) of the radial bores (26, 27).

6. Head clamp according to claim 5, **characterised in that** the locking lever (35), the fork arms (33, 34) of which are connected together by a transverse web (36) arranged above the holding arm (3), is provided with a handle part (39), which can be fixed in the locking position of the locking lever (35) by means of a securing device (84).

7. Head clamp according to claim 6, **characterised in that** the handle part is designed as a transverse web (39) connecting the ends of the fork arms (33, 34), whereby in the movement path of the transverse web a locking slide (84) of the securing device can be introduced in a locking manner.

8. Head clamp according to claim 4 or 5, **characterised in that** the two fork arms (33, 34) of the locking lever (35) on an arc (45) concentric to its pivot axis (30) comprise mutually aligned recesses (43, 44), into which the balls (24, 25) can partly dip to release the threaded spindle (10).

9. Head clamp according to claim 8, **characterised in that** the recesses consist of bores (43, 44), the diameter of which is smaller than the diameter of the identical balls (24, 25).

10. Surgical head clamp according to claim 1, **characterised in that** the insertion bore (54) is located in a head part (51) of the guiding shaft (50) forming a radial annular shoulder (52) for a compression spring (53), opposite which guiding shaft lies a radial annular shoulder (63) of an axial bore (61) of the threaded spindle (10), and **in that** the guiding shaft (50) is provided at its end lying opposite the mounting bore with a stop element (66) delimiting its flexible axial movement.

11. Surgical head clamp according to claim 1 or 10, **characterised in that** for mounting a multiple arm pin support (12, 13) with several holding pins (14) onto the guiding shaft (50) an insertion pin (55) is provided, which has an insertion projection (56) fitting into the insertion bore (54), which insertion pin on the end face has a sphere-like depression (70) for pivotably and rotatably mounting a multiple arm pin holder (12, 13) provided with a suitable counter profile.

12. Surgical head clamp according to claim 11, **characterised in that** for pivotably securing the multiple arm pin support (12, 13) a ball head screw (73) fitting the counter profile is screwed into a central threaded bore (74) of the insertion bolt (55) and is fixed by means of a locking screw (75).

## Revendications

1. Serre-tête chirurgical, constitué d'un étrier de maintien (1) essentiellement en forme de U ayant deux branches de support (3, 4) s'étendant aux extrémités d'une partie de liaison (2) et dans le plan de celle-ci vers le même côté, branches dont les extrémités (5, 6) présentent des supports de pointes (11) qui sont montés à déplacement dans des paliers mutuellement coaxiaux et qui peuvent être pourvus d'une ou plusieurs pointes de maintien (14), sachant qu'un premier support de pointe est fixé à pivotement sur un élément formant palier (15), mobile autour d'un axe de palier commun (9), de la première extrémité de branche (6), et qu'un deuxième support de pointe (11) est mobile au moyen d'une broche filetée (10) dans un alésage de palier (7) de l'autre extrémité de branche (5),
**caractérisé en ce que** le deuxième support de pointe (11) est constitué d'une tige de guidage (50) qui est pourvue frontalement d'un alésage d'emboîtement (54) et qui est montée à ressort, coaxialement à l'axe de palier (9), dans un alésage axial (61) de la broche filetée (10), et **en ce que** la broche filetée (10), pourvue d'un filetage extérieur, est montée à déplacement dans un alésage de palier cylindrique et lisse (7) de l'autre extrémité de branche (5), sachant que le filetage extérieur est réalisé sous forme de filetage à billes (23) avec un profil du genre segment de cercle dans lequel s'engagent comme éléments d'engagement deux billes (24, 25) qui sont disposées dans la paroi (28) de l'alésage de palier (7) en étant diamétralement opposées et axialement immobiles, et qui sont maintenues en engagement avec le filetage à billes (23) dans cette position de blocage au moyen d'un levier de verrouillage réglable (35), et qui dans une autre position du levier de verrouillage (35) peuvent être désengagées de la broche filetée (10) afin de rendre la broche filetée (10) librement coulissante dans son alésage de palier (7).

2. Serre-tête chirurgical selon la revendication 1, **caractérisé en ce que** l'alésage de palier (7) est en outre pourvu d'une bille de crantage (81) s'engageant radialement à ressort dans le filetage à billes (23).

3. Serre-tête chirurgical selon la revendication 1, **caractérisé en ce que** les billes (24, 25) sont respectivement logées dans l'un de deux alésages radiaux mutuellement coaxiaux (26, 27) de parties de paroi (28) de l'alésage de palier (7) recevant en ajustement la broche filetée (10), sachant que les épaisseurs (d) des parties de paroi (28) sont respectivement inférieures au diamètre des billes.

4. Serre-tête selon la revendication 2, **caractérisé en ce que** la branche de support (3) présente au moins dans la région des alésages radiaux (26, 27) deux faces extérieures plates, parallèles et planes (41, 42) s'étendant perpendiculairement à l'axe (29) desdits alésages et contre lesquelles est disposé à pivotement un levier de verrouillage (35) à deux branches dont les branches de fourche (33, 34) peuvent recouvrir les alésages radiaux (26, 27) respectivement dans le plan d'une des faces de délimitation (41, 42).

5. Serre-tête selon la revendication 4, **caractérisé en ce que** le levier de verrouillage (35) présente deux branches de fourche (33, 34) ayant des faces intérieures (31, 32) parallèles et planes qui s'appliquent contre les faces extérieures (41, 42) de la branche de support (3) et qui sont montées sur la branche de support (3) à pivotement autour d'un axe de pivotement excentrique (30) parallèle à l'axe (29) des alésages radiaux (26, 27).

6. Serre-tête selon la revendication 5, **caractérisé en ce que** le levier de verrouillage (35), dont les branches de fourche (33, 34) sont mutuellement reliées par une entretoise (36) située au-dessus de la branche de support (3), est pourvu d'une partie de préhension (39) qui, dans la position de blocage du levier de verrouillage (35), peut être immobilisée au moyen d'un organe d'arrêt (84).

7. Serre-tête selon la revendication 6, **caractérisé en ce que** la partie de liaison est réalisée sous la forme d'une entretoise (39), qui relie les extrémités des branches de fourche (33, 34) et dans la voie de déplacement de laquelle peut être introduit, pour la bloquer, un coulisseau de blocage (84) de l'organe d'arrêt.

8. Serre-tête selon la revendication 4 ou 5, **caractérisé en ce que** les deux branches de fourche (33, 34) du levier de verrouillage (35) présentent des évidements mutuellement alignés (43, 44), qui sont situés sur un arc de cercle (45) concentrique à l'axe de pivotement (30) desdites branches et dans lesquels les billes (24, 25) peuvent partiellement s'enfoncer pour libérer la broche filetée (10).

9. Serre-tête selon la revendication 8, **caractérisé en ce que** les évidements consistent en des alésages (43, 44) dont le diamètre est inférieur au diamètre des billes (24, 25) identiques entre elles.

10. Serre-tête chirurgical selon la revendication 1, **caractérisé en ce que** l'alésage d'emboîtement (54) se trouve dans une partie de tête (51) de la tige de guidage (50), partie qui forme un épaulement annulaire radial (52) pour un ressort de pression (53) et à laquelle fait axialement face un épaulement annulaire radial (63) d'un alésage axial (61) de la broche filetée (10), et **en ce que** la tige de guidage (50) est pourvue, à son extrémité faisant face à l'alésage récepteur, d'un élément de butée (66) limitant son mouvement axial à ressort.

11. Serre-tête chirurgical selon la revendication 1 ou 10, **caractérisé en ce que,** afin de monter sur la tige de guidage (50) un porte-pointes (12, 13) à plusieurs bras, pourvu de plusieurs pointes de maintien (14), il est prévu un boulon à emboîtement (55) qui est pourvu d'un embout d'emboîtement (56) s'ajustant dans l'alésage d'emboîtement (54) et qui présente frontalement un renfoncement du genre calotte (70) pour le montage à pivotement et à rotation d'un porte-pointes (12, 13) à plusieurs bras doté d'un profil complémentaire adapté.

12. Serre-tête chirurgical selon la revendication 11, **caractérisé en ce que,** pour la fixation à pivotement du porte-pointes (12, 13) à plusieurs bras, une vis à tête sphérique (73) adaptée au profil complémentaire est vissée dans un alésage fileté central (74) du boulon à emboîtement (55) et immobilisée au moyen d'une contre-vis (75).
